# EUROPEAN PATENT APPLICATION

(11) **EP 1 780 272 A1**
(43) Date of publication of application: **02.05.2007**
(21) Application number: 05023503.5
(22) Date of filing: 27.10.2005
(51) Int. Cl.: C12N 15/00

(54) **Method for enhancing somatic hypermutation, gene conversion and class switch recombination**

(71) Applicant: GSF-Forschungszentrum für Umwelt und Gesundheit GmbH, 85764 Neuherberg (DE)
(72) Inventor: Buerstedde, Jean-Marie, Dr., 80807 München (DE); Schötz, Ulrike, 84048 Mainburg (DE)
(74) Representative: UEXKÜLL & STOLBERG

(57) **Abstract**

The invention relates to a method for enhancing somatic hypermutation, gene conversion and/or immunoglobulin class switch recombination in a cell by increasing the level of an E2A encoded protein in the cell. The invention also relates to cells and cell lines with an increased level of said activities.

## Description

The present invention relates to a method for enhancing somatic hypermutation, gene conversion, and/or class switch recombination in a cell by increasing the level of an E2A encoded protein in the cell.

Lately a number of genetic systems has been developed which allow to diversify a particular target sequence in a cell by exploiting such phenomena as gene conversion and somatic hypermutation. WO 00/22111 and WO 02/100998 describe a human Burkitt lymphoma cell line (Ramos) which is capable of directed constitutive hypermutation of a specific nucleic acid region.

WO 02/100998 also describes another cell system for generating genetic diversity in the immunoglobulin (Ig) locus which is based on the chicken B cell line DT40 containing deletions of the paralogues of the RAD51 gene which are involved in homologous recombination and DNA repair.

In EP 1568765 the present inventors describe a gene diversification system in which gene conversion can be combined with or substituted by somatic hypermutation.

The described systems are based on a phenomenon which takes place in lymphoid cells. B cells diversify the rearranged V(D)J segments within their light and heavy chain Ig loci by frequent nucleotide substitutions leading to the emergence of antibodies with increased antigen affinity (Neuberger et al., 2003). Ig hypermutation distinguishes itself from other known hypermutation states by being limited to a region of about 2 kb downstream of the Ig gene transcription start sites (Peters and Storb, 1996). Although transcription of the Ig genes is required for hypermutation, the vast majority of all transcribed genes of B cells do not accumulate mutations at detectable frequency. Nevertheless, mutations, most likely due to deregulated hypermutation activity, have been found in some non-Ig genes of B cells (Gordon et al., 2003). Analysis of artificial transgenes in hypermutating B cells indicates that the Ig promoter can be replaced by other promoters without an effect on hypermutation, but that some of the Ig enhancer sequences are indispensable for the mutation activity (Betz et al., 1994; Storb and al., 1999).

The only known B cell specific factor which plays a role in gene conversion and somatic hypermutation is activation-induced cytidine deaminase (AID). The present inventors and others demonstrated that Ig hypermutation as well as Ig gene conversion and switch recombination require expression of the AID gene (EP 1568765; Muramatsu et al., 2000; Revy et al., 2000; Arakawa et al., 2002; Harris et al., 2002). AID is believed to initiate hypermutation and recombination by the deamination of cytidines in the Ig loci (DiNoia and Neuberger, 2002).

Overexpression of AID in non-B cells could induce somatic hypermutation in artificial constructs inserted in various loci in the genome (Kotani et al., 2005). Somatic hypermutation and class switch recombination was also induced by AID in non-mutating B cell hybridomas (Martin et al., 2002). Surprisingly, AID expression in fibroblasts leads to a high mutation rate in non-Ig transgenes (Yoshikawa et al., 2002), suggesting that the restriction of hypermutation to the Ig loci is B cell specific or related to chromatin configuration. It was proposed that AID introduces mutations in a wide variety of genes with little specificity (Kotani et al., 2005). Thus, it appears that although the phenomenon of gene conversion and somatic hypermutation is restricted to B cells and the Ig loci under normal conditions, it can be artificially induced or modulated in a variety of cells and loci.

Thus, the need existed in the prior art for further factors apart from AID which are involved in the regulation of gene conversion, somatic hypermutation and immunoglobulin class switch recombination. The need also existed for improved cell systems for genetic diversification of target nucleic acids.

The present invention satisfies these needs by providing a further factor, an E2A encoded protein, and a method for enhancing gene conversion, somatic hypermutation and immunoglobulin class switch recombination in a cell.

The E2A encoded protein is known as a transcription factor. It serves multiple functions in B cell commitment and differentiation most likely by regulating the transcription of other genes (reviewed in Kee et al., 2000). The avian E2A gene has been cloned and functionally characterized (Conlon and Meyer, 2004). The introduction of an artificial E-box DNA sequence, to which the E2A encoded protein E47 as well as many other transcription factors can bind, was shown to increase hypermutation in an artificial transgene (Michael et al., 2003). However, the prior art provides no evidence for a role of E2A in genetic recombination and mutation.

### SUMMARY OF THE INVENTION

In the first aspect of the invention there is provided a method of introducing or enhancing gene conversion and/or somatic hypermutation and/or immunoglobulin class switch recombination in a cell by increasing the level of an E2A encoded protein in the cell. The level of an E2A encoded protein can be increased by increasing the expression of the endogenous E2A gene or by providing the cell with an exogenous E2A gene or protein. In particular, the recombination and mutation activities in the cell may depend on the presence of AID and/or limited to a particular genetic locus such as, for example, the immunoglobulin locus. The locus may contain one or a few E-box binding sites and/or a target nucleic acid which is subjected to genetic diversification by somatic hypermutation or gene conversion.

The cell can be artificially provided with additional factors which affect recombination and/or mutation activities. For example, these factors can be cis-acting regulatory sequences such as an immunoglobulin promoter and enhancer sequences. In a preferred embodiment, the levels of the AID and the E2A encoded proteins are increased in the cell to achieve an optimal level of gene conversion and/or somatic hypermutation and/or immunoglobulin class switch recombination activity at defined target loci without an increase in the mutation rate in other parts of the genome.

In a second aspect, the invention relates to a cell obtained as a result of the method of the invention. The cell of the invention may possess gene conversion and/or somatic hypermutation and/or immunoglobulin class switch recombination activity before the level of an E2A encoded protein is increased. Alternatively, these activities may be acquired after the level of an E2A encoded protein is increased in the cell. Preferably, the cell is a lymphoid cell, or most preferably a DT40 cell.

In a third aspect, the invention relates to a cell line derived from the cells of the invention and to a transgenic non-human vertebrate containing such cells.

In a further aspect, the invention relates to the use of an E2A gene or an E2A encoded protein for producing a cell or a vertebrate with an enhanced gene conversion and/or somatic hypermutation and/or class switch recombination activity.

### DESCRIPTION OF THE FIGURES

**Figure 1. E2A gene disruption**
   (A) Aligned maps of the chicken E2A locus, the targeting constructs and the disrupted locus after targeted integration of the constructs and marker excision.
   (B) Flow chart showing the generation of the E2A overexpressing, knockout and re-complemented clones.
**Figure 2. sIgM expression analysis of E2A negative, E47 reconstituted, E12 overexressing and control clones**
   (A) FACS anti-IgM staining profiles of representative subclones derived from initially sIgM(+) clones. AID^{-/-}ψV⁻ is an AID knock-out DT40 clone used as a negative control for Ig hypermutation. AID^{R}ψV⁻ is an AID positive (reconstituted) pseudo-V negative clone used as a progenitor clone for the study of Ig hypermutation. AID^{R}ψV⁻E2A^{+/-} is a heterozygous E2A knock-out clone. AID^{R}ψV⁻E2A^{-/-} Cl.1 and Cl.2 are homozygous E2A knock-out clones. AID^{R}ψV⁻E2A^{E12R} and AID^{R}ψV⁻E2A^{E47R} are derived from the E2A knock-out clone AID^{R}ψV⁻E2A^{-/-} Cl.2 by transfection of the E12 and E47 cDNA expression constructs, respectively. AID^{R}ψV⁻tE12 is derived from the AID^{R}ψVprogenitor clone by transfection of the E12 cDNA expression construct.
   (B) Average percentages of events falling into sIgM(-) gates.

### DETAILED DESCRIPTION OF THE INVENTION

The present invention provides a particularly useful method for enhancing such genetic activities as somatic hypermutation, gene conversion and immunoglobulin class switch recombination in a cell.

"Hypermutation" refers to the mutation of a nucleic acid in a cell at a rate above background. Preferably, hypermutation refers to a rate of mutation of between 10⁻⁵ and 10⁻³ bp⁻¹ generation⁻¹. This is greatly in excess of background mutation rates, which are of order of 10⁻⁹ to 10⁻¹⁰ mutations bp⁻¹ generation⁻¹ (Drake et al. 1991).

"Gene conversion" refers to a phenomenon in which sequence information is transferred in unidirectional manner from one homologous allele to the other. Gene conversion may be the result of a DNA polymerase switching templates and copying from a homologous sequence, or the result of mismatch repair (nucleotides being removed from one strand and replaced by repair synthesis using the other strand) after the formation of a heteroduplex.

"Immunoglobulin class switch recombination" or "isotype switch recombination" refers to a site-specific recombination phenomenon which takes place in B cells and results in the exchange of the constant region within Ig genes in response to certain stimuli. For example, the first immunoglobulin isotype produced after B cell stimulation, IgM, will later change to IgG, IgE or IgA.

Hypermutation and gene conversion generate natural diversity within the immunoglobulin V(D)J segment of B cells. Hypermutation takes place in the germinal centers of such species as mouse and human following antigen stimulation. Gene conversion takes place in primary lymphoid organs like the Bursa of Fabricius or the gut-associated lymphoid tissue in such species as cow, rabbit, sheep and swine independent of antigen stimulation. In chicken, stretches from the upstream pseudo-V-genes (ΨV) are transferred into the rearranged V(D)J segment.

In the present invention, a factor is provided which enhances gene conversion, somatic hypermutation and immunoglobulin class switch recombination in a cell. This factor is a protein encoded by the E2A gene. More specifically, this factor is an E47 or E12 protein encoded by alternatively spliced E2A mRNAs.

The recombination and mutation activities in a cell can be enhanced by increasing the level of an E2A encoded protein in the cell. The E2A level can be increased by increasing the expression of the endogenous E2A gene, or by providing the cell with an artificial E2A gene expression construct, or with the protein itself. The E2A gene or its splice variant can be permanently or conditionally expressed. In the latter case, E2A can be expressed under a drug-responsive promoter such as the tetracycline responsible gene expression system or gene expression may be shut down by e.g. the Cre recombinase-mediated excision of an loxP flanked E2A expression cassette. Switching off the E2A expression will decrease the rate or prevent further diversification of target nucleic acids in the cell. Preferably, E2A expression is switched off in the cell producing a gene product with a desired activity in order to prevent further mutations which can lead to the loss of the desired activity. In a specific embodiment, the level of the AID protein in the cell is also regulated, either prior to the up-regulation of the E2A protein or simultaneously with the E2A protein.

Gene conversion, somatic hypermutation or class switch recombination can be regulated in a variety of cells. In a preferred embodiment, the regulation takes place in cells which are originally capable of gene conversion, somatic hypermutation and/or class switch recombination. Preferred cells are thus lymphoid cells, in particular B cells or cell lines. The chicken B cell line DT40 is preferred because it allows efficient genetic manipulation, i.e. it allows to insert transfected constructs into a particular genomic locus by targeted integration at a high frequency (Buerstedde and Takeda, 1991). Moreover, DT40 continues gene conversion of the rearranged light chain immunoglobulin gene during cell culture (Buerstedde et al., 1990).

In EP 1568765 the present inventors showed that gene conversion can be partially or fully substituted by somatic hypermutation. Thus, in a preferred embodiment a B cell line is provided which exhibits elevated rates of somatic hypermutation due to an increased level of an E2A encoded protein in the cell. This cell line will efficiently diversify a target nucleic acid and produce the desired protein. For example, antibodies can be produced with an optimized affinity to a particular antigen.

Alternatively, gene conversion, somatic hypermutation and class switch recombination activities can be induced in cells which originally do not possess these activities by increasing the level of an E2A encoded protein.

Gene conversion, somatic hypermutation and class switch recombination can be further enhanced or optimized by providing the cell with additional factors influencing these activities. These factors can be genetic elements which are known to be involved in the regulation of genetic recombination and/or mutation activities, such as immunoglobulin promoter and enhancer sequences. Alternatively, these factors can be proteins such as AID. A combination of genetic and protein factors is included in the invention.

Gene conversion, somatic hypermutation or class switch recombination can be limited to a particular genetic locus such as the light or heavy chain immunoglobulin locus. However, genetic diversification of other loci by gene conversion and somatic hypermutation is also included in the invention. Particularly preferred are genetic loci which contain one or more putative E2A binding sites such as E-box binding site. One preferred E-box motif is CAGGTG. Alternatively, E2A binding sites can be artificially introduced into a locus of interest.

The genetic locus may contain a target nucleic acid which is subjected to genetic diversification.

A "target nucleic acid" is a nucleic acid sequence or chromosomal region in the cell according to the present invention which is subjected to genetic diversification by somatic hypermutation or gene conversion. The target nucleic acid can be either endogenous or transgenic and may comprise one or more transcription units encoding gene products.

The target nucleic acid may encode a protein or possess a regulatory activity. Examples of proteins are an immunoglobulin chain, a selection marker, a DNA-binding protein, an enzyme, a receptor protein or a part thereof. In a preferred embodiment, the target nucleic acid is the V(D)J segment of a rearranged human immunoglobulin gene. Examples of regulatory nucleic acids are a transcription regulatory element, an RNAi sequence, or a splice acceptor site.

The invention also provides a transgenic non-human vertebrate species having an increased level of an E2A encoded protein. The vertebrates of the invention can be used as a source of peptides, polypeptides or proteins having a desired property. The desired property is a result of genetic diversification of a target nucleic acid in the vertebrate cells with an enhanced gene conversion and/or somatic hypermutation activity. It is preferred that the desired peptides, polypeptides and proteins can be easily isolated from the vertebrate, for example from the blood, milk or eggs. The preferred vertebrates are chicken, mice, cows and sheep.

The invention is illustrated by the following example.

### EXAMPLE

Immunoglobulin (Ig) hypermutation is limited to a region of about 2 kilo bases (kb) downstream of the transcription start sites of the Ig loci. The process requires transcription and the presence of Ig enhancer sequences and is most likely initiated by AID mediated deamination of cytidine bases.

In this example it is demonstrated that the inactivation of the E2A gene strongly reduces the rate of Ig light chain mutations in the B cell line DT40 without affecting the levels of surface Ig (sIg) or AID expression. The defect is complemented by the expression of cDNAs corresponding to either of the two E2A splice variants, E12 and E47. The overexpression of the E12 cDNA in the wild-type E2A cells leads to a significant increase in the hypermutation rate as compared to the parental cells. The results suggest that E2A encoded proteins enhance Ig hypermutation by recruitment of AID to the Ig loci. As Ig gene conversion and class switch recombination are also initiated by AID, it is expected that E2A enhances these processes as well.

### Materials and Methods

### Construction of the E2A knockout constructs and cDNA expression vectors

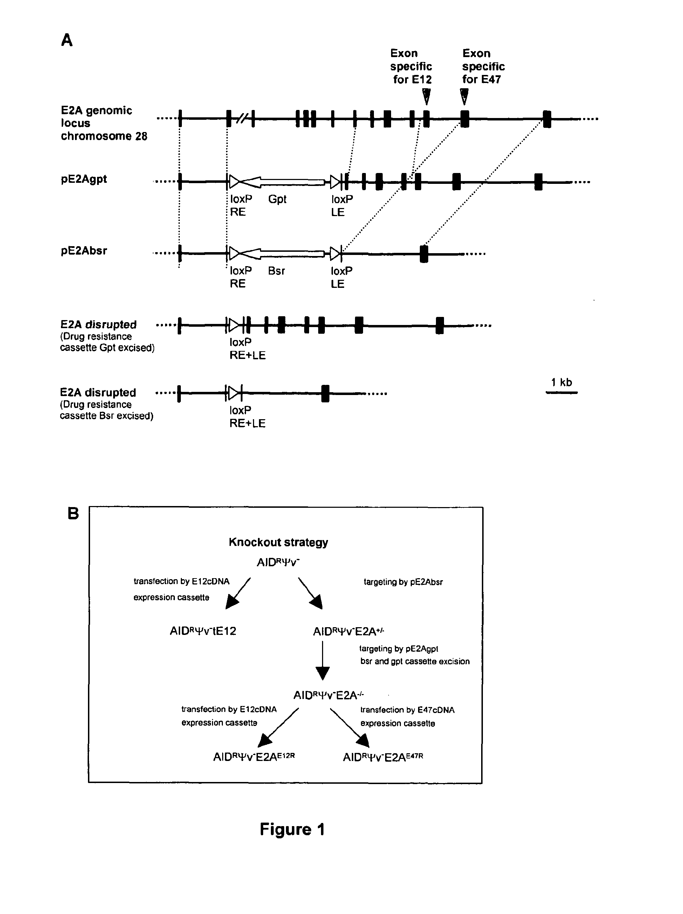

The full-coding E12 and E47 cDNAs and the exon-intron structure of the E2A locus has been described previously (Conlon and Meyer, 2004). The target arms for the E2A knockout constructs were amplified by long-range PCR using genomic DNA from DT40 as template (Figure 1A). The primer included restriction sites attached at the 5' end to facilitate cloning of the PCR fragments. The arms were cloned upstream and downstream of the floxed drug resistant marker cassettes (Arakawa et al., 2001). The constructs were linearised by NotI before transfection. E12 and E47 full length cDNAs were isolated from the riken1 bursal cDNA library (Caldwell et al., 2005) and their entire sequence was verified by primer walks. The E12 and E47 expression constructs were made by cloning the corresponding cDNAs downstream of the (β-actin promoter and upstream of an IRES-bsr sequence.

### Generation of the E2A knock-out clones

The pE2Absr construct was transfected into the AID^{R}ψV⁻ clone and blasticidin resistant colonies having integrated the construct by targeted integration were identified using a primer derived from the E2A locus upstream of the 5' target arm and a primer derived from the drug resistance marker. Targeting efficiency was about 20%. The transfection of a heterozygous E2A disruption mutant by a targeting construct which differed from pE2Absr only by including the gpt gene instead of the bsr gene did not yield homozygous E2A disrupted clones. Therefore, a new construct was made which specifically targets the wild-type allele of the heterozygous E2A mutant. Transfection of this construct yielded the homozygous E2A disrupted mutants AID^{R}ψV⁻E2A^{-/-} Cl.1 and Cl.2.

### Cell culture

Cell culture, transfection, selection of stable transfectants and marker recycle by transient Cre induction were performed as previously described (Arakawa et al., 2002).

### Ig reversion assays and sequence analysis

Quantification of sIgM expression by fluorescence activated cell sorting (FACS) and sequence analysis of light chain VJ regions were described previously (Arakawa et al., 2004).

### Results

### Disruption of E2A and complementation by E12 and E47 cDNA expression

The 5' and 3' arms of the E2A knockout constructs were designed to delete exons encoding evolutionary conserved domains of E2A proteins (Figure 1A). The mutations introduced upon targeted integration of the constructs are therefore most likely null mutations. The DT40 variant AID^{R}ψV⁻ (Arakawa et al., 2004) was chosen as the progenitor clone of the study, because it diversifies its rearranged Ig light chain locus by hypermutation, expresses AID under an artificial promoter and can be induced for Cre recombinase. Stepwise transfections of the E2A knock-out constructs, identification of targeted transfectants and the subsequent excision of the drug resistant marker cassettes yielded the homozygous E2A mutant clones, AID^{R}ψV⁻E2A^{-/-} Cl.1 and AID^{R}ψV⁻E2A^{-/-} Cl.2 (Figure 1B). To test for genetic complementation, AID^{R}ψV⁻E2A^{-/-} Cl.2 was transfected either by an E12 or an E47 cDNA expression vector. The transfection yielded the clones AID^{R}ψV⁻E2A^{El2R} and AID^{R}ψV⁻E2A^{E47R}, respectively.

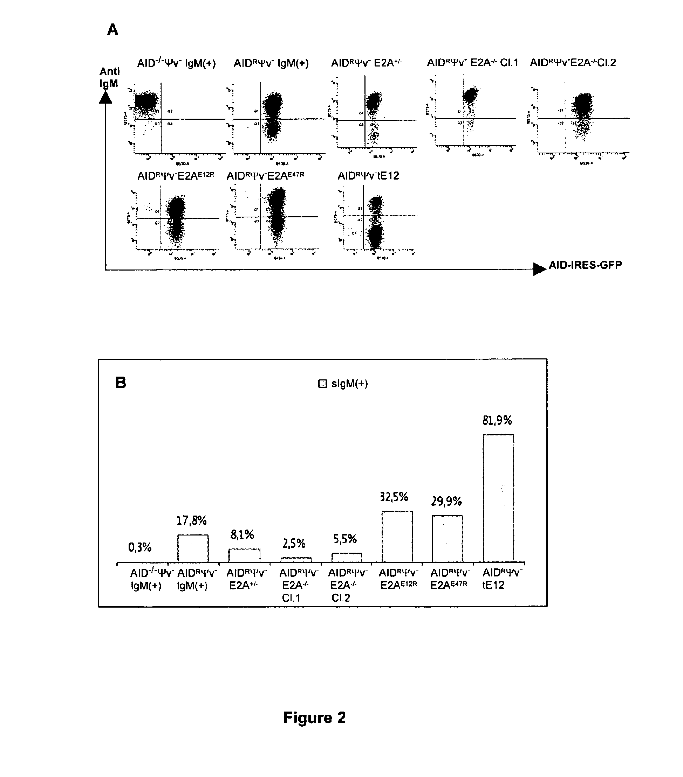

FACS analysis after staining with anti-IgM antibody showed that the sIgM(+) cells of all clones displayed similar sIgM levels (Figure 2A) indicating that E2A is not needed for efficient transcription of the Ig genes in DT40.

### E2A negative clones display decreased rates of sIgM loss.

All clones included in the study were predominantly sIgM(+) and the rate of Ig light chain mutations can be estimated by measuring the appearance of sIgM(-) populations which are due to deleterious mutations in the light chain VJ segment (Arakawa et al., 2004). The appearance of sIgM(-) subpopulations was measured in parallel in at least 24 subclones of the following clones: AID^{-/-}ψV⁻, AID^{R}ψV⁻, AID^{R}ψV⁻E2A^{+/-}, AID^{R}ψV⁻E2A^{-/-} Cl.1 and Cl.2, AID^{R}ψV⁻E2A^{E12R} and AID^{R}ψV⁻E2A^{E47R} (Figures 2A and 2B). The analysis revealed a reduction in the percentages of sIgM(-) cells for subclones of the heterozygous E2A mutant clone AID^{R}ψV⁻E2A^{+/-} (8.1%) and the homozygous E2A mutant clones AID^{R}ψV⁻E2A^{-/-} Cl.1 and Cl.2 (2.5% and 5.5%) compared to subclones of the wild-type AID^{R}ψV⁻ cells (17.8%) and of the complemented E2A mutant clones AID^{R}ψV⁻E2A^{E12R} (32.5%) and AID^{R}ψV⁻E2A^{E47R} (29.9%). The stable sIg(+) control clone AID^{-/-}ψV⁻ which has stopped all Ig hypermutation due to the deletion of the AID cDNA expression cassette showed only few events in the sIg(-) gates (0.3%).

### Overexpression of the E12 splice variant in the E2A wild-type cells leads to a strongly increased rate of sIgM loss

The high percentages of sIg(-) cells of the E2A complemented subclones suggest that increased levels of E2A encoded proteins increase Ig hypermutation above the level seen in wild-type E2A cells. To further study the effect of E2A overexpression upon Ig hypermutation, AID^{R}ψV⁻ cells containing the wild-type E2A gene were transfected with the E12 cDNA expression vector. FACS analysis of one of the resulting clones, AID ^{R}ψV⁻tE12 revealed a strong increase in the percentage of sIgM(-) cells (81.9%) compared to the wild-type AID^{R}ψV⁻ cells (17.8%) (Figures 2A and 2B).

### Discussion

We report here that the inactivation of the E2A gene strongly reduces Ig hypermutation without affecting Ig expression levels. The reduction is independent on AID expression levels which should be maintained constant under an artificial promoter in all clones used. Expression of either the E12 or the E47 cDNA under a strong promoter not only complements the Ig hypermutation defect in E2A disrupted cells, but increases the hypermutation rates above the wild-type level. This is the first report that an Ig enhancer binding transcription factor influences Ig hypermutation independent of Ig transcription and AID expression.

The E2A encoded proteins E12 and E47 are known transcription factors which recognise an E-box consensus sequence in the loci of target genes. As E-box sequence motives are present in the enhancers of Ig and other hypermutating genes (Kotani et al., 2005), the most plausible scenario is that E12 and E47 influence Ig hypermutation by binding to the Ig enhancers. This is also consistent with the observation that the introduction of an E-box sequence stimulated the mutation rate of a transgene (Michael et al., 2003). It remains unknown, how binding of the E2A encoded proteins to the Ig enhancers stimulates Ig hypermutation. However, it is tempting to speculate that the effect is either due to AID recruitment or the stimulation of AID cytidine deaminase activity. Since E2A most likely works by recruiting AID to the Ig loci, it can be assumed that it enhances not only Ig hypermutation, but also Ig gene conversion and class switch recombination.

### REFERENCES

Arakawa, H., Lodygin, D., and Buerstedde J. M. (2001). Mutant loxP vectors for selectable marker recycle and conditional knock-outs. BMC Biotechnol. 1: 7.

Arakawa, H., Hauschild, J., Buerstedde, J. M. (2002). Requirement of the activation-induced deaminase (AID) gene for immunoglobulin gene conversion. Science 295: 1301-1306.

Arakawa, H., Saribasak, H., Buerstedde J. M. (2004). Activation-induced cytidine deaminase initiates immunoglobulin gene conversion and hypermutation by a common intermediate. PLoS Biol. 2:967-974.

Betz, A. G. , Milstein, C., Gonzalez-Fernandez, A., Pannell, R., Larson, T., Neuberger, M. S. (1994). Elements regulating somatic hypermutation of an immunoglobulin kappa gene: critical role for the intron enhancer/matrix attachment region. Cell 77:239-48.

Buerstedde, J. M. and Takeda, S. (1991). Increased ratio of targeted to random integration after transfection of chicken B cell lines. Cell 67(1):179-88.

Buerstedde, J-M. et al (1990). Light chain gene conversion continues at high rate in an ALV-induced cell line. EMBO J. 9: 921-927.

Caldwell, R. B., Kierzek, A. M., Arakawa, H., Bezzubov, Y., Zaim, J., Fiedler, P., Kutter, S., Blagodatski, A., Kostovska, D., Koter, M., Plachy, J., Carninci, P., Hayashizaki, Y., Buerstedde, J. M. (2005). Full-length cDNAs from chicken bursal lymphocytes to facilitate gene function analysis. Genome Biol. 6:R6.

Conlon, T. M. and Meyer, K. B. (2004). Cloning and functional characterisation of avian transcription factor E2A. BMC Immunol. 5: 11.

Di Noia, J., Neuberger, M. S. (2002). Altering the pathway of immunoglobulin hypermutation by inhibiting uracil-DNA glycosylase. Nature 419:43-8*.*

Drake, J. W. (1991). Spontaneous mutation. Annu Rev Genet. 25:125-46.

Gordon, M. S., Kanegai, C. M., Doerr, J. R., Wall R. (2003). Somatic hypermutation of the B cell receptor genes B29 (Igbeta, CD79b) and mbl (Igalpha, CD79a). Proc Natl Acad Sci U S A 100:4126-31.

Harris, R. S., Sale, J. E., Petersen-Mahrt, S. K., and Neuberger, M. S. (2002). AID is essential for immunoglobulin V gene conversion in a cultured B cell line. Curr Biol. 12: 435-438.

Kee, B. L., Quong, M. W., Murre C. (2000). E2A proteins: essential regulators at multiple stages of B-cell development. Immunol Rev 175:138-49.

Kotani, A., Okazaki, I., Miramatsu, M., Kinoshita, K., Begum, N., A., Nakajima, T., Saito, H., Honjo T. (2005). A target selection of somatic hypermutation is regulated similarly between T and B cells upon activation-induced citidine deaminase expression. Proc Natl Acad Sci U S A 102:4506-4511.

Martin, A., Bardwell, P. D., Woo, C. J., Fan, M., Shulman, M. J., Sharff, M. D. (2002). Activation-induced cytidine deaminase turns on somatic hypermutation in hybridomas. Nature 415: 802-806.

Michael, N., Shen, H. M., Longerich, S., Kim, N., Longacre, A., Storb, U. (2003). The E box motif CAGGTG enhances somatic hypermutation without enhancing transcription. Immunity 1:235-42.

Muramatsu, M., Kinoshita, K., Fagarasan, S., Yamada, S., Shinkai, Y. and Honjo, T. (2000). Class switch recombination and hypermutation require activation-induced cytidine deaminase (AID), a potential RNA editing enzyme. Cell 102: 553-563.

Neuberger, M. S., Harris, R. S., Di Noia, J., Petersen-Mahrt, S.K. (2003). Immunity through DNA deamination. Trends Biochem Sci. 28:305-12.

Peters, A. and Storb, U. (1996). Somatic hypermutation of immunoglobulin genes is linked to transcription initiation. Immunity 4:57-65.

Revy, P., Muto, T., Levy, Y., Geissmann, F., Plebani, A., Sanal, O., Catalan, N., Forveille, M., Dufourcq-Labelouse, R., Gennery, A., Tezcan, I., Ersoy, F., Kayserili, H., Ugazio, A. G., Brousse, N., Muramatsu, M., Notarangelo, L. D., Kinoshita, K., Honjo, T., Fischer, A., Durandy, A. (2000). Activation-induced cytidine deaminase (AID) deficiency causes the autosomal recessive form of the Hyper-IgM syndrome (HIGM2). Cell 102:565-75.

Storb, U., Peters, A., Kim, N., Shen, H. M., Bozek, G., Michael, N., Hackett, J. Jr., Klotz, E., Reynolds, J. D., Loeb, L. A., Martin, T. E. (1999). Molecular aspects of somatic hypermutation of immunoglobulin genes. Cold Spring Harb Symp Quant Biol. 64:227-34.

Yoshikawa, K., Okazaki, I. M., Eto, T., Kinoshita, K., Muramatsu, M., Nagaoka, H., Honjo, T. (2002). AID enzyme-induced hypermutation in an actively transcribed gene in fibroblasts. Science 14:2033-6.

## Claims

1. Method of introducing or enhancing gene conversion and/or somatic hypermutation and/or immunoglobulin class switch recombination in a cell by increasing the level of an E2A encoded protein in the cell.

2. The method of claim 1, wherein the cell is capable of gene conversion and/or somatic hypermutation and/or immunoglobulin class switch recombination before the level of an E2A encoded protein is increased in the cell.

3. The method of claim 1 or 2, wherein the level of an E2A encoded protein is increased by increasing the expression of the endogenous E2A gene or a splice variant thereof.

4. The method of claim 1 or 2, wherein the level of an E2A encoded protein is increased by providing the cell with an exogenous E2A gene or a splice variant thereof.

5. The method of claim 1 or 2, wherein the level of an E2A encoded protein is increased by providing the cell with an exogenous protein encoded by an E2A gene or a splice variant thereof.

6. The method of claims 1 to 5, wherein gene conversion and/or somatic hypermutation and/or class switch recombination depends on the presence of activation-induced cytidine deaminase (AID).

7. The method of claims 1 to 6, wherein gene conversion and/or somatic hypermutation and/or class switch recombination is limited to a particular genetic locus.

8. The method of claim 7, wherein the genetic locus contains one or a few putative E-box binding sites.

9. The method of claim 7 or 8, wherein the genetic locus contains a target nucleic acid.

10. The method of claims 7 to 9, wherein the genetic locus is an immunoglobulin locus.

11. The method of claims 1 to 10, wherein the level of activation-induced cytidine deaminase (AID) is increased in the cell.

12. The method of claims 1 to 11, wherein the cell is provided with genetic elements involved in the regulation of gene conversion, somatic hypermutation and/or immunoglobulin class switch recombination.

13. A genetically modified cell produced according to the method of claims 1 to 12.

14. The cell of claim 13, wherein the cell is a lymphoid cell.

15. The cell of claim 14, wherein the cell is a DT40 cell.

16. A cell line derived from the cell according to claims 13 to 15.

17. A transgenic non-human vertebrate containing the cells according to claims 13 to 15.

18. The transgenic non-human vertebrate of claim 17, wherein the non-human vertebrate is a mouse, a chicken, a sheep, a goat, a rat, a rabbit, a swine or a cow.

19. Use of an E2A gene or an E2A encoded protein *in vitro* for producing a cell with an enhanced gene conversion and/or somatic hypermutation and/or class switch recombination activity.

20. Use of an E2A gene or an E2A encoded protein for producing a transgenic non-human vertebrate with an enhanced gene conversion and/or somatic hypermutation and/or immunoglobulin class switch recombination activity.
